# EUROPEAN PATENT SPECIFICATION

(11) **EP 0 883 601 B1**
(45) Date of publication and mention of the grant of the patent: **23.08.2000**
(21) Application number: 96935156.8
(22) Date of filing: 04.11.1996
(51) Int. Cl.: C07C 235/20, C07C 235/24, C07D 273/00

(54) **CALIXARENES AND THEIR USE FOR SEQUESTRATION OF METALS**
CALIXARENE UND IHRE VERWENDUNG FÜR DAS ABTRENNEN VON METALLEN
CALIXARENES ET LEUR UTILISATION POUR LA SEQUESTRATION DE METAUX

(30) Priority: 10.11.1995 GB 9523119
(43) Date of publication of application: 16.12.1998
(73) Proprietor: THE SECRETARY OF STATE FOR DEFENCE IN HER BRITANNIC MAJESTY'S GOVERNMENT OF THE UNITED KINGDOM OF GREAT BRITAIN, London SW1A 2HB (GB)
(72) Inventor: NICHOLSON, Graeme, Peter, Chemical Analysis Branch, Reading RG7 4PR (GB); KAN, Mark, Joseph, Chemical Analysis Branch, Reading RG7 4PR (GB); WILLIAMS, Gareth, Chemical Analysis Branch, Reading RG7 4PR (GB); DREW, Michael, George, Chemistry Department, Reading RG6 2AD (GB); BEER, Paul, Derek, Inorganic Chemistry Laboratory, Oxford OX1 3QR (GB)
(74) Representative: Skelton, Stephen Richard
(86) International application number: GB9602687
(87) International publication number: WO9717322

(56) References cited:
- EP-A- 0 432 989
- WO-A-95/01346
- JOURNAL OF THE CHEMICAL SOCIETY, PERKIN TRANSACTIONS 2, no. 1, 1995, LETCHWORTH GB, pages 131-4, XP002023154 D. M. RUDKEVICH: "Calix[4]arene-triacids as receptors for lanthanides; synthesis and luminescence of neutral Eu3+ and Tb3+ complexes"
- ANGEWANDTE CHEMIE INTERNATIONAL EDITION., vol. 29, no. 3, 1990, WEINHEIM DE, pages 280-282, XP002023155 M. A. MCKERVEY: "A new type of double calix[4]arenes by linkage via the phenolic hydroxy group"
- JOURNAL OF THE CHEMICAL SOCIETY, PERKIN TRANSACTIONS 1, 1991, LETCHWORTH GB, pages 3137-42, XP002023156 E. M. COLLINS ET AL.: "Chemically modified calix[4]arenes. Regioselective synthesis of 1,3-(distal) derivatives and related compounds. X-ray crystal structure of a diphenol dinitrile"

## Description

The present invention relates to novel calixarenes, methods of their preparation, and uses thereof, in particular for the sequestration of metals.

International publication number WO 95 01346 discloses calix[4]arenes with three substituent carboxylic acid groups and a substituent carboxamido group. Two such molecules were linked together using the carboxamido group to form dimer species. *J*. *Chem. Soc. Perkins Trans. 2,* 1995, p.131 disclosed further examples of analogous calix[4]arenes with three carboxylic acid groups and one carboxamido group, the calix[4]arenes so disclosed being capable of ligating metal ions, especially lanthanides.

European Patent Publication No. 0 432 989 describes a number of calixarene and oxacalixarene derivatives as having metal sequestering properties, and reviews some of the prior art in this field.

In a first aspect of the present invention there is disclosed calixarenes of the formula (I). The term calixarene as used hereinafter is intended to embrace also oxacalixarenes, 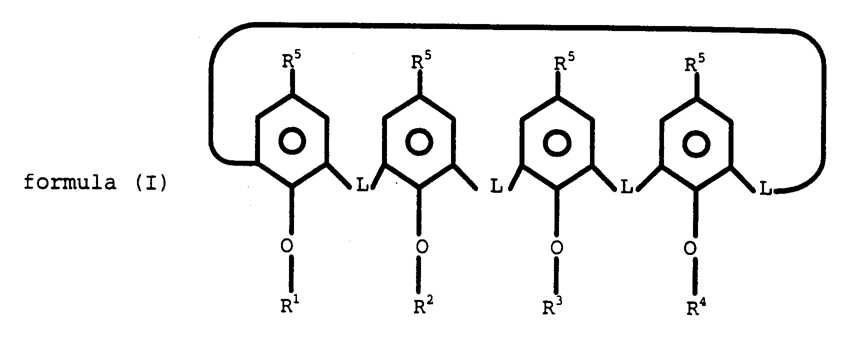 wherein:
L is [-CH₂-] or [-O-CH₂-O-] and may be the same or different between each aryl group.
R⁵ is H, halogen, or C₁ - C₁₀ aliphatic hydrocarbyl group, C₆ - C₂₀ aryl group, C₆ - C₂₀ hydrocarbylaryl group, any of which may optionally be substituted by one or more halo or oxo groups or interrupted by one or more oxo groups, and R⁵ may be the same or different on each aryl group.
R¹ comprises a carboxy group which may or may not be protonated or protected. Suitable protecting derivatives include salts and ester derivatives of the carboxylic acid.
two groups out of R², R³, and R⁴ are H
the one group out of R², R³, and R⁴ not being H comprises an amide. group

The combination of 'acid' (or protected acid) and 'amide' in the calixarenes of the present invention is not found in the calixarenes of the prior art; this combination leads to unexpected and desirable metal sequestering properties (particularly for lanthanide and actinide cations) as will be further discussed below.

Preferably:
R² and R⁴ are H and R³ comprises the amide group; L is [-CH₂-]- between each of the aryl groups;
R⁵ is tertiary alkyl, especially butyl.

Preferably the carboxy group R¹ conforms to the general formula (A):

(A) [ -X-COOR¹⁰]

wherein X is a C₁, a C₂ or a C₃ carbon chain being a part of an aliphatic hydrocarbyl group, aryl group or hydrocarbylaryl group, any of which may optionally be substituted by one or more halo, oxo or nitro groups.
R¹⁰ is H or a protecting group being a salt or an ester derivative. Salts include metal salts e.g. alkali (such as Li) or alkali earth metals, or ammonium or substituted ammonium derivatives. The choice of salt should be made such as to prevent the cation interfering with the operation of the calixarene in practice. Ester groups may be formed with C₁ - C₁₀ aliphatic alkyl alcohols, C₆ - C₂₀ aryl alcohols, C₆ - C₂₀ hydrocarbylaryl alcohols, any of which may optionally be substituted by one or more halo, nitro, or oxo groups or interrupted by one or more oxo groups. Examples include benzyl, p-methoxybenzyl, benzoylmethyl, p-nitrobenzyl, methyl, ethyl, butyl, t-butyl etc.

More preferably R¹ is of the general formula (B) :

(B) [ -(C.R⁶.R⁷)ₙ-COOR¹⁰]

wherein n is 1, 2 or 3 and R⁶ and R⁷ are H or halogen and can be the same or different on each carbon.

Alternatively R¹ may be of the general formula (C): 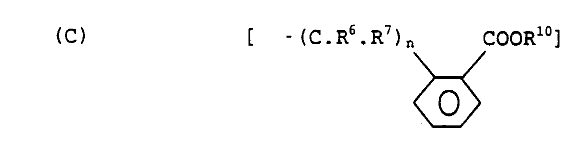 wherein n is 0 or 1 and R⁶ and R⁷ are H or halogen and can be the same or different on each carbon and wherein the phenyl ring of the benzoic acid group may be optionally substituted by one or more halo, oxo or nitro groups.

In each case it is preferable that n is 1 and R⁶, R⁷ and R¹⁰ are all H.

In unprotected acid embodiments, preferably the aliphatic hydrocarbyl group, aryl group or hydrocarbylaryl group of X in formula (A) are substituted by one or more groups which cause a reduction in the pKa of the carboxy group with respect to the unsubstituted molecule e.g. nitro.

For instance the phenyl ring of the benzoic acid of formula (C) is preferably substituted by one or more groups which cause a reduction in the pKa of the carboxy group with respect to the unsubstituted molecule e.g. nitro.

Preferably the amide group R², R³, or R⁴ of formula (I) is of the general formula (D): 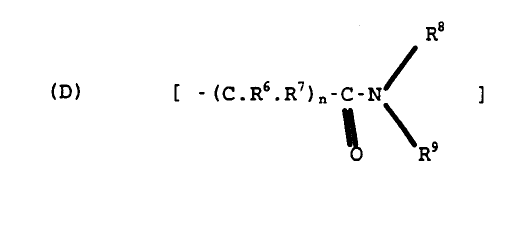 wherein n is 1, 2 or 3 and R⁶ and R⁷ are H, halogen, or C₁ - C₁₀ aliphatic hydrocarbyl group, and can be the same or different on each carbon, and wherein R⁸ and R⁹, which may be the same or different, are H or C₁ - C₁₀ aliphatic hydrocarbyl group (optionally halo substituted) including a cycloaliphatic ring formed by R⁸ and R⁹ together.

In certain embodiments of the invention, as described in more detail below, R⁸ or R⁹ may form a bridge between a calixarene of the present invention and a further calixarene in order to produce a dimer.

Most preferably, the calixarene is of the formula (II): 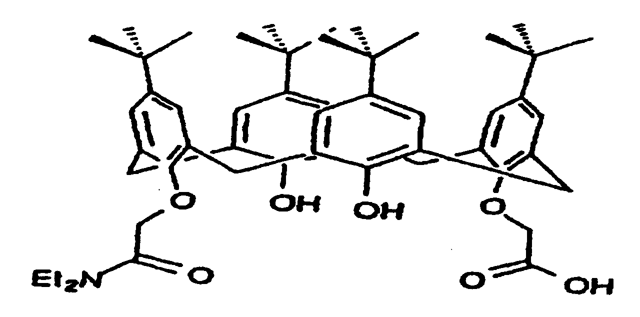 (5,11,17,23-tetra-tert-butyl-25-[hydroxycarbomylmethoxy] -27-[(N-diethylamino) carbomylmethoxy]-26-28-dihydroxy-calix[4]arene.)

This compound ("acid-amide") has been found to be useful for the extraction of both divalent and trivalent metal ions such as Pb, Sr, Hg, Bi and Y; in particular Lanthanides (e.g. La) and Actinides (e.g. U).

Also embraced by the present invention are calixarenes of the general formulae (I) and (II) but wherein some or all of phenyl groups of the calixarene ring are further peripherally substituted in such a way as not to compromise the advantageous combination of the carboxy and amide groups which form the central core of the present invention. Possible substituents include halogen, nitro, C₁ - C₁₀ aliphatic hydrocarbyl group, C₆ - C₂₀ aryl group, or C₆-C₂₀ hydrocarbylaryl group, any of which may optionally be substituted by one or more halo or oxo groups or interrupted by one or more oxo groups. Indeed certain substituents (e.g. nitro) may be desirable in as much as they reduce the pKa values of the two hydroxy groups of the calixarene ring, thereby modifying the metal-chelating properties of the compound.

In a second aspect of the present invention there is disclosed a method of sequestering metals comprising contacting the metals with a calixarene as described above.

Preferably the calixarene is used to complex metals at a pH of 2-6, (most preferably pH 3-6) since at higher pHs there is an increased risk of the target metal precipitating. For instance, precipitation of Lanthanides occurs at fairly low pH (7.5 for La, 6.4 for Lu).

If required, additional complexing agents (such as are well known to the skilled person) may be used to prevent precipitation of target metals. This allows the use of the calixarene at higher pHs, which will advantageously reduce protonation of the carboxy and hydroxy groups. The use of such additional complexing agents can thus raise the useful working pH range of the calixarene to the point at which the metal-calixarene complex itself precipitates e.g. around pH 11.

The use of higher pHs (e.g. pH 7 to 10, preferably pH 9) may be particularly advantageous for increasing the concentration of negative charge in calixarenes having protected acid groups or in calixarene-dimers, which may otherwise be reduced by the protecting group or steric effects respectively.

If desired the environmental pH may be adjusted using conventional methods of the art. For instance if it is desired to raise the pH, then LiOH may be added. If desired, the pH may be buffered by using an appropriate buffer such as are well known to those skilled in this art e.g. citrate.

In all cases the lower pH limit of useful operation will be dependent on the pKa of each chelating group in the calixarene, since that will dictate whether each (unprotected) carboxy or hydroxy group will be protonated at any given pH. It may therefore be desirable for each group to have a low pKa e.g. when treating acidic waste streams for which the pH cannot be readily adjusted. The pKa of the protonated carboxy and the amide group of the calixarene of formula (II) is less than 3.

Preferably the calixarene is dissolved in a hydrophobic organic solvent (e.g. dichloromethane) and this is mixed with an aqueous phase containing metal ions (e.g. in equal volumes).

The phases are then stirred or otherwise agitated, typically for around 1 hour, followed by a 2 hour separation time.

Preferably the calixarene is present in excess over the metal target e.g. 25-fold, or 250-fold. The excess required for useful extraction will depend on the nature of the metal target e.g. size, charge etc.

Preferably the metal target is U, Hg, Am, Pb, Sr, Bi, or Y for instance in methods of environmental clean up. Alternatively the metal could be an actinide such as Am or another lanthanide.

The calixarenes described above are such that the metal complexes formed with the target ion may be overall neutral without the necessity for additional counter-anions. A further advantage is that the calixarenes can be highly selective, thereby preventing unwanted metal ions complexing all available sites.

A still further advantage of the methods of the current invention is that the extracted metal ions can be recovered following sequestration into the hydrophobic phase simply by contacting that phase with a relatively small (with respect to the original metal-containing sample) volume of acid (e.g. 1 M) thereby causing the pH to drop and the metal to become decomplexed and enter the acid aqueous phase. The calixarene can then be reused simply by evaporation of the solvent.

Alternatively, the extracted metal ions can be recovered following extraction simply by evaporating the solvent to leave the metal-calixarene complex.

Thus in preferred forms, e.g. using the 'acid-amide' above, the extraction methods of the present invention are both selective and efficient and do not require additional ions to operate. The nature of the extraction can be readily optimised by adjustment of the pH.

In a third aspect of the invention there is disclosed a solid phase-bound calixarene of the type described above e.g. polymer bound. For instance the calixarene may be physisorbed and immobilised onto polystyrene divinyl benzene beads. Immobilisation of the calixarene on a solid phase support may assist in the extraction methods of the invention. The preparation of such bound calixarenes would present no undue burden to those skilled in the art, in the light of the present disclosure in conjunction with the methods, or methods analogous to the methods, described by Harris *et al*. in US 4,642,362 or 4,699,966, or Parker in US 4,447,585 or Tetrahedron 36 461-510 (1980), or in European Patent Publication No. 0 217 656.

In a fourth aspect of the invention there is disclosed a process for preparing the calixarenes described above. Intermediates for use in the process form a fifth aspect of the invention.

In a sixth aspect of the invention there is disclosed a calixarene dimer comprising two calixarenes of formula (I) wherein the amide group of each is of the general formula (D) above, and wherein the R⁸ or R⁹ group of one calixarene is conjugated to the R⁸ or R⁹ of the other calixarene, optionally through a spacer group R¹¹, as shown schematically in formula (III):

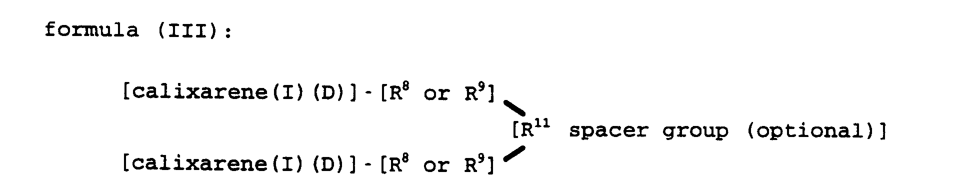

The optional spacer group R¹¹ may be C₁ - C₆ aliphatic hydrocarbyl group, C₆ - C₁₀ aryl group, C₆ - C₁₆ hydrocarbylaryl group, any of which may optionally be substituted by one or more halo or oxo groups or interrupted by one or more oxo groups. In the absence of a spacer group the R⁸ or R⁹ group of one calixarene is conjugated directly to the R⁸ or R⁹ group of the other. In any case it is preferable that there is only 1, 2, 3 or 4 bridging atoms (preferably carbon atoms) between the Nitrogen atoms of the two amide groups. Most preferably there is 2 or 3 bridging carbon atoms. As described in more detail below, this spacing between the calixarenes may help to pre-stress the dimer into a particular stable, low-energy, chelating conformation, and thereby enhancing the specificity for target metals with respect to calixarene monomers.

The compounds, methods and processes of the present invention will now be described, by way of illustration only, through reference to the following Figures and Examples. Other embodiments falling within the scope of the invention will occur to those skilled in the art in the light of these.

### FIGURES

Fig 1 shows the acid-amide of the present invention.

Fig 2 shows the efficiency of extraction of La(III) by acid-amide as a function of concentration ratio of the two.

Fig 3 shows the efficiency of extraction of La(III) by acid-amide as a function of the presence of various anions (citrate, acetate, picrate).

Fig 4 shows the efficiency of extraction of La(III) by acid-amide as a function of concentration of buffer (citrate).

Fig 5 shows the efficiency of competitive extraction of different Lanthanide(III) cations by acid-amide in the presence of buffer (citrate).

Fig 6 shows the efficiency of extraction of various metals by acid-amide in the presence of buffer (citrate).

Fig 7 shows the efficiency of extraction of various metals by acid-amide in the absence of buffer.

Fig 8 shows the structure of an acid-amide/Ln(III) complex, as determined by X-ray crystallography.

Fig 9 shows the structure of an acid-amide/Lu(III) complex, as determined by X-ray crystallography.

Fig 10 shows a calixarene dimer (designated 13b) according to the present invention.

Fig 11 shows a calixarene dimer (designated llb) according to the present invention, having an aryl spacer group between the Nitrogen atoms of the two amide groups.

Fig 12 shows a calixarene dimer (designated 10) according to the present invention wherein the carboxy groups of the calixarenes have been protected by esterification with benzyl alcohol. The Nitrogen atoms of the two amide groups are linked via a 2-C ethyl bridge. The tertiary group of the Nitrogens (designated R⁹) is methyl in each case.

Fig 13 shows a calixarene dimer (designated 11) according to the present invention wherein the carboxy groups of the calixarenes have been protected by esterification with benzyl alcohol. The Nitrogen atoms of the two amide groups are linked via a 2-C ethyl bridge. The tertiary group of the Nitrogens (designated R⁹) is hydrogen in each case.

Fig 14 shows a calixarene dimer (designated 11a) according to the present invention, wherein the carboxy groups of the calixarenes have been protected by as an ethyl ester. The Nitrogen atoms of the two amide groups are linked via a 3-C aromatic bridge. The tertiary group of the Nitrogens is hydrogen in each case.

Fig 15 shows a synthetic scheme for the acid-amide (954)

Fig 16 shows a synthetic scheme for the azacrown-acid calix[4]arenes A957 and A959

### EXAMPLES

EXAMPLE 1 - The pH changes associated with the combination of various of the agents used in the later examples was first measured in order to better interpret the findings. The standard extraction of dichloromethane and aqueous phase in equal volumes with 1 hour stirring plus 2 hours separation was employed. The La(III) was used at a concentration of 0.4 mM, and the other agents were used in a ratio of 1:3:24 for La(III):citrate:acid-amide. The results, measured to +/- 0.1 pH units, are shown in Table 1.

**Table 1**

| Solution | pH before | pH after |
|---|---|---|
| type | extraction | extraction |
| (no agents) | 5.6 | 5.5 |
| acid-amide | 5.6 | 5.6 |
| La(III) | 5.6 | 4.9 |
| acid-amide La(III) | 5.6 | 4.0 |
| Citrate | 6.0 | 6.1 |
| Citrate acid-amide | 6.0 | 6.1 |
| Citrate La(III) | 6.0 | 6.0 |
| Citrate acid-amide La(III) | 6.0 | 6.1 |

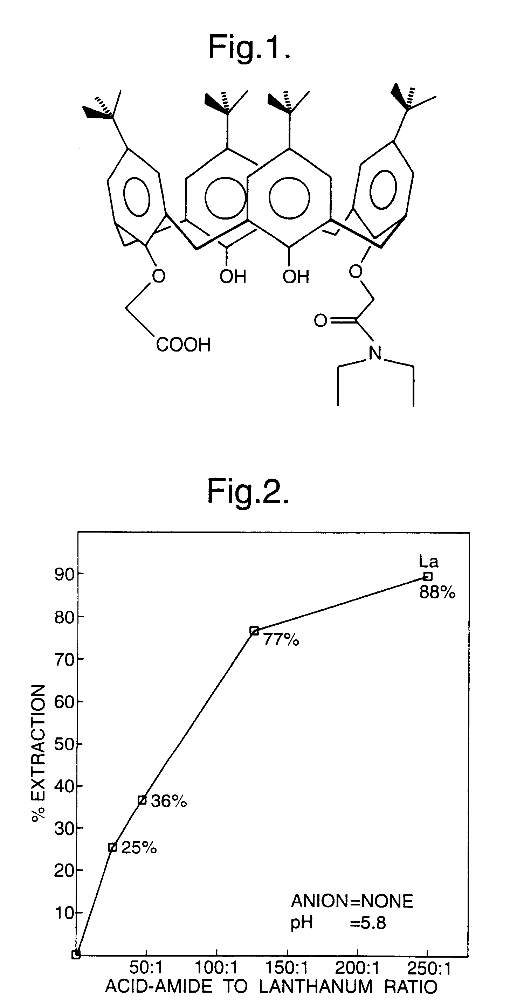

EXAMPLE 2 - The efficiency of extraction of La(III) by acid-amide as a function of the concentration ratio of the two was measured at an initial pH of 5.8 (Fig 2). The pH was not maintained at this level during the experiment. A result of 90% extraction was achieved using a large (250 x) excess of acid-amide. It is postulated that this large excess was required because of a drop in pH during the course of the experiment (see Example 1) which led to reduced deprotonation of the three ionizable groups. In a similar experiment using UO₂²+ only a 25 x excess was required, possibly because as a divalant cation it can still be efficiently bound when the three ionizable groups of the acid-amide are partially protonated

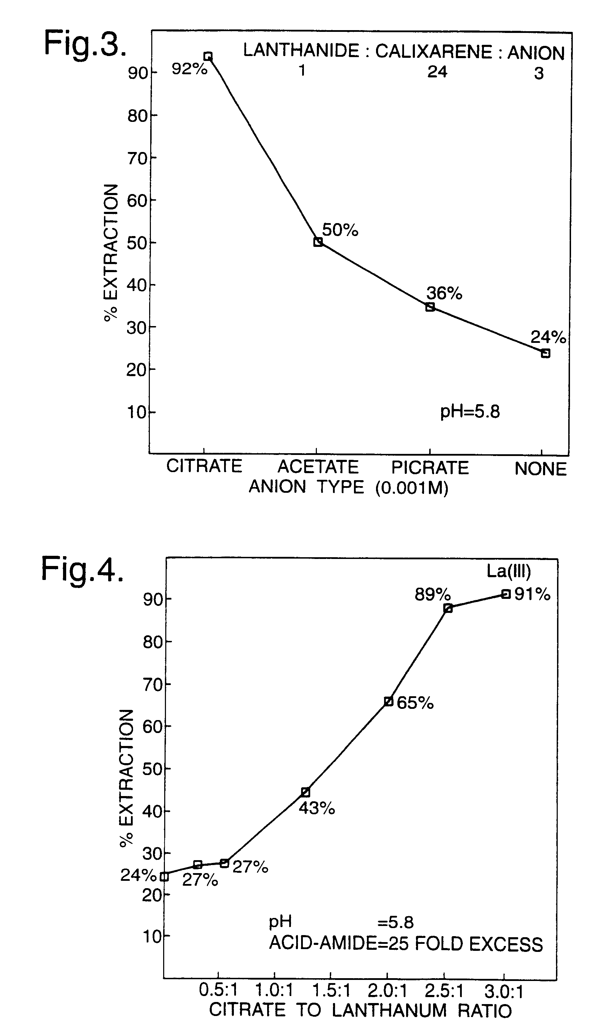

EXAMPLE 3 - The effect of various anions on the efficiency of extraction is shown in Fig 3. Citrate was found to be the best, probably because of its buffering ability. In order to demonstrate that citrate is not itself involved in the actual extraction or complexation of La(III), LiOH was titrated into the mixture to retain pH 6 instead of using a citrate buffer. The level of extraction obtained (90% La(III)) was similar to that achieved with citrate, indicating that citrate is not actually required to achieve efficient acid-amide extraction. The postulated non-coordination of the La(III) by citrate when acid-amide is present indicates a high formation constant (i.e. tight binding) for the La(III)/acid-amide complex.

EXAMPLE 4 - The optimum amount of citrate required for La(III) extraction was assessed (Fig 4). The results indicate that a 3x excess over La(III) is suitable.

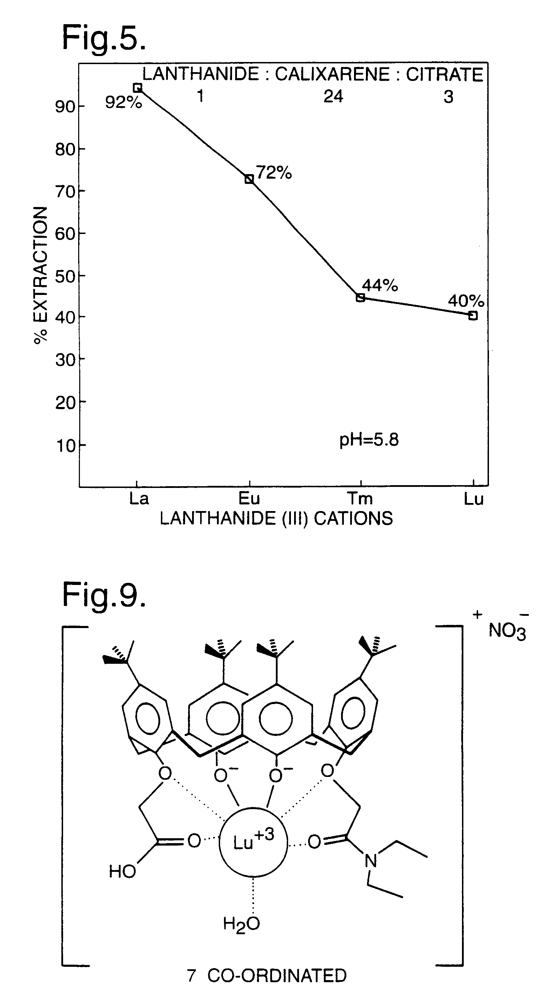

EXAMPLE 5 - The efficiency of competitive extraction of various members of the Lanthanide series is shown in Fig 5. The efficiency appears to drop off across the series, probably as a result of the change in the size of the metal cations. The results with Lanthanides indicate that it is likely certain actinides such as Am(III) will also be efficiently extracted.

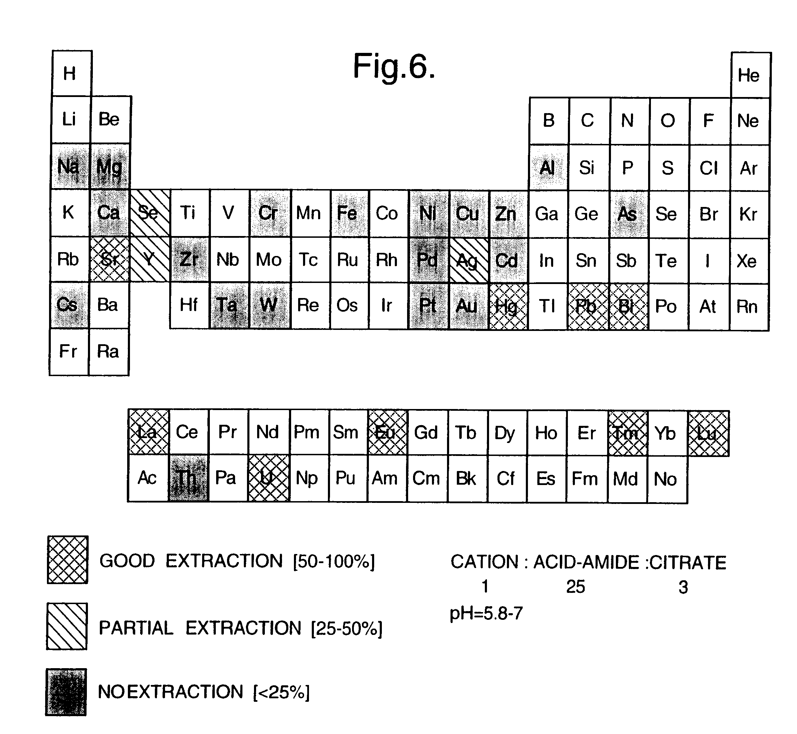

EXAMPLE 6 - The efficiency of extraction of various metals by acid-amide in the presence of citrate was measured, the results being shown in Fig 6. The results indicate high selectivity within the broad range of elements assessed. The extraction of La, U, Hg, Sr, Eu, Tm, Lu, Bi, and Pb is especially efficient, particularly as compared with the alkali and the smaller alkali-earth metals, and various other transition metals.

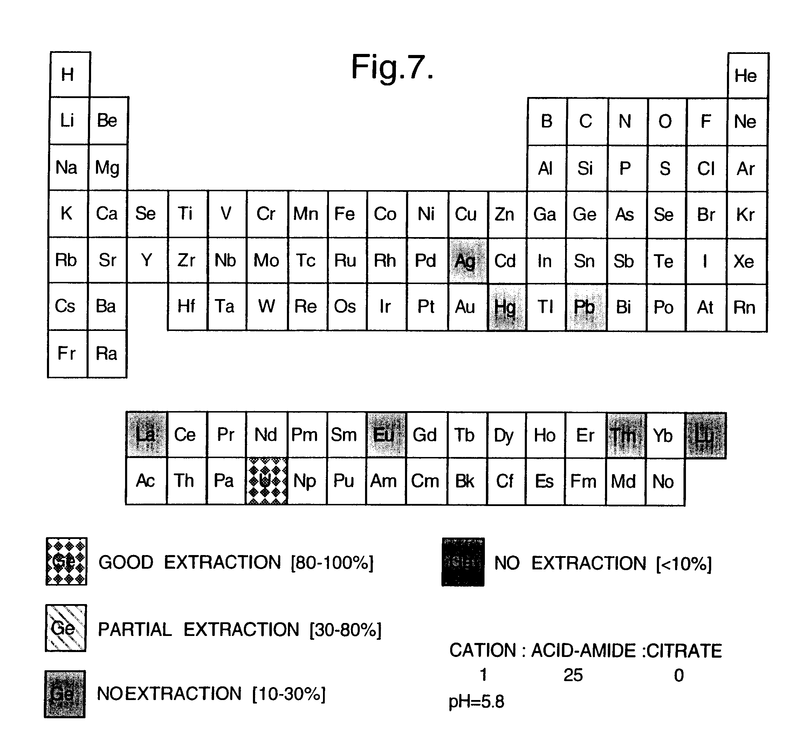

EXAMPLE 7 - Fig 7 shows the efficiency of extraction of various metals by acid-amide in the absence of buffer. As can be seen, efficiency is reduced as compared with Fig 6 (with buffer).

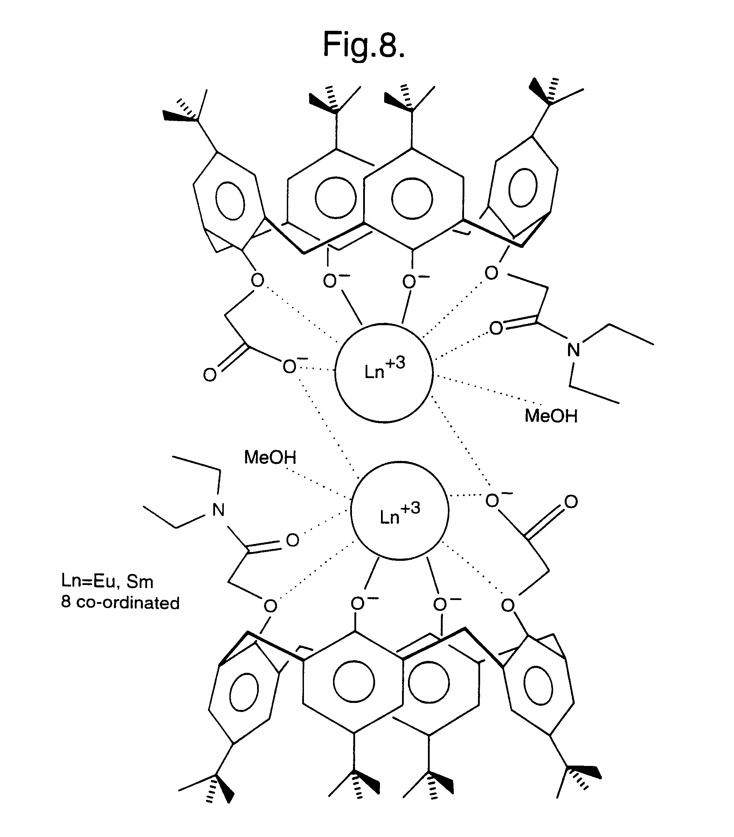

EXAMPLE 8 - Single crystals of some metal/acid-amide complexes (Sm, Eu, Lu) were grown and analysed using X-ray crystallography. Results indicate that the intermediate Lanthanides (Sm, Eu) prefer to form a neutral dimer structure of 2 acid-amide molecules binding 2 metal ions (see Fig 8 which shows an acid-amide/Ln(III) complex, wherein Ln = Sm or Eu). The complex is a dimer in the solid state. The acid-amide takes up the cone conformation. The Sm cations are 8 coordinate, being bound to the deprotonated phenolic oxygen atoms, the ethereal oxygen atoms, the amide oxygen and one of the carboxyl oxygens. The remaining two coordination sites are made up from a methanol oxygen and a carboxyl oxygen from the second calixarene hence forming a bridge between the two calixarenes.

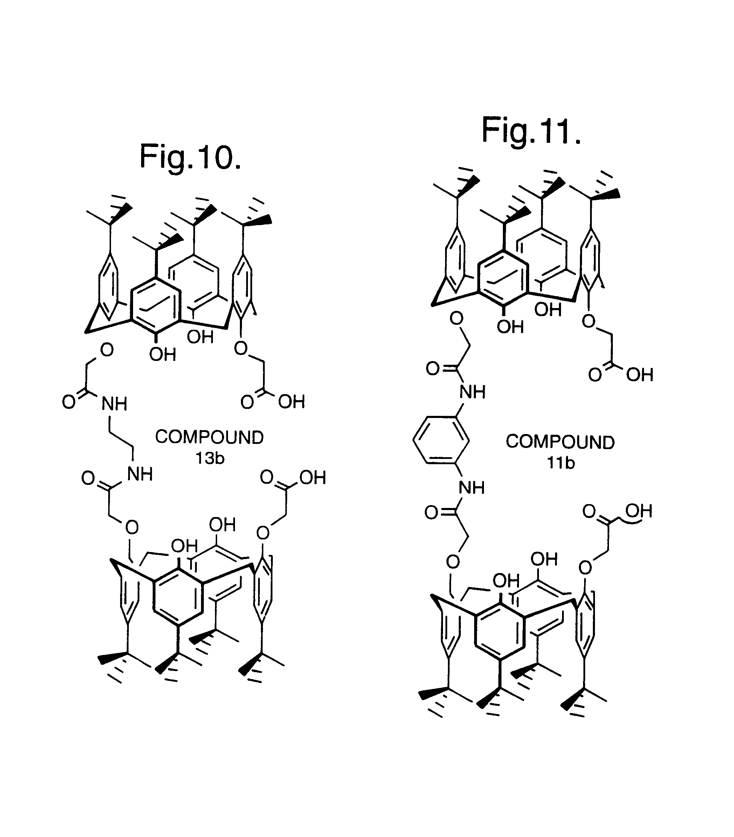

Molecular modelling suggested that all the larger Lanthanides would form isomorphic structures and that only the smaller Lanthanides (Gd-Lu) would form discrete monomeric complexes. Lu (smallest Lanthanide) forms a structure with 1 acid-amide and 1 metal ion which requires a counter anion for charge neutrality. Fig 9 shows an acid-amide/Lu(III) complex with NC₃⁻ as the counter ion. The Lu cation is shown to be seven coordinate, bound to the two phenolate oxygens, the two ethereal oxygens, the amide oxygen, one carboxylate oxygen and a water molecule.

These structures may help to account for the specificity demonstrated in Examples 5 and 6.

EXAMPLE 9 - Metal/acid-amide complexes were further investigated by extracting the complexes from the hydrophobic phase and determining the metal:acid-amide ratio. For La, Lu and U at pH 6 the M:L ratio was 1:1. This confirms the solid-state ratios determined for the larger lanthanides and Lu by X-ray crystallography in Example 8 (which were 2:2 and 1:1 respectively). No X-ray data was obtained for U.

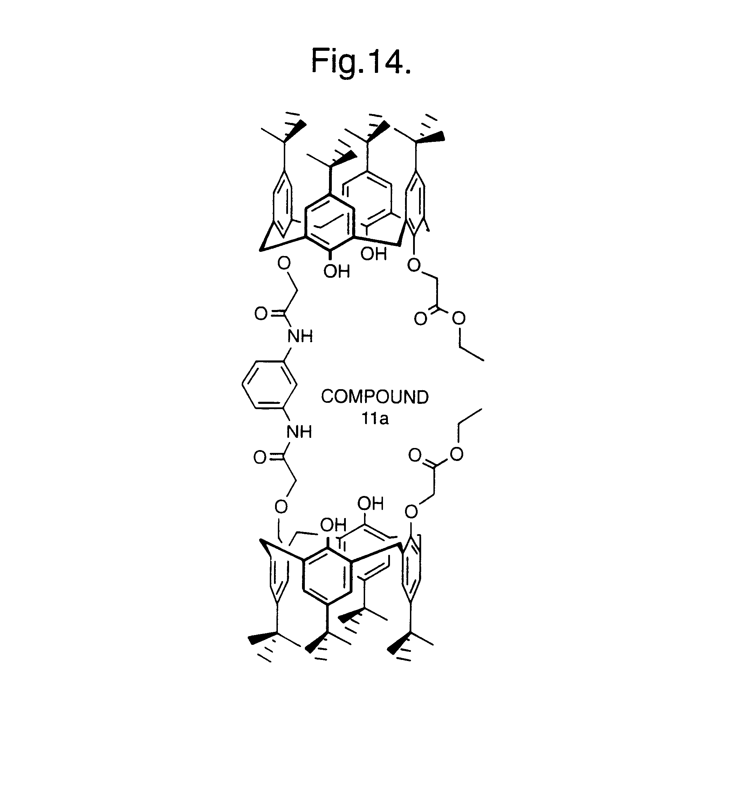

EXAMPLE 10 - Acid-amide dimers and esters thereof were prepared based on the acid-amide calixarenes of the present invention, as described in more detail in Example 15 below. Some of these are shown Figures 10 to 14.

**Compound 13b** (Figure 10) was prepared in order to mimic the calixarene/Lanthanide complex of Figure 8. The dimer did not complex La³⁺ at pH 6, a more alkaline pH (i.e. pH 9) being required to quantitatively extract La. This is possibly because steric hindrance may reduce La's ability to compete with protons for oxygen coordination sites at low pHs. Metal:Ligand ratios in the solvent extracted complex were determined to be 0.54 i.e. for every 2 La:dimer. This suggests that all six ionizable -OH groups are dissociated forming a complex similar to that in Figure 8. La, in the presence of Lu and U, at pH 9 is preferentially extracted.

By contrast, U is quantitatively extracted at pH 6 (unlike La). The Metal:Ligand ratio at pH 6 was approximately 1:1 suggesting a different complex is forming to that formed by La at higher pH.

**Compound 11b** (Figure 11) was prepared in order to optimise the bridging group between the calixarenes for U extraction. The meta-di-phenylamine linkage restricts the two calixarene halves such that the carboxyl groups are close to each other. This is the predicted conformation in the metal complex, unlike the conformation in free solution, wherein it is predicted that steric effects will mean that the halves are diametrically opposed around the bridging group. The compound extracted U much more efficiently at pH 9 than pH 6 (80% rather than 20%). This is in contrast to Compound 13b above. The more alkaline operating conditions of llb may be more applicable to some clean up applications.

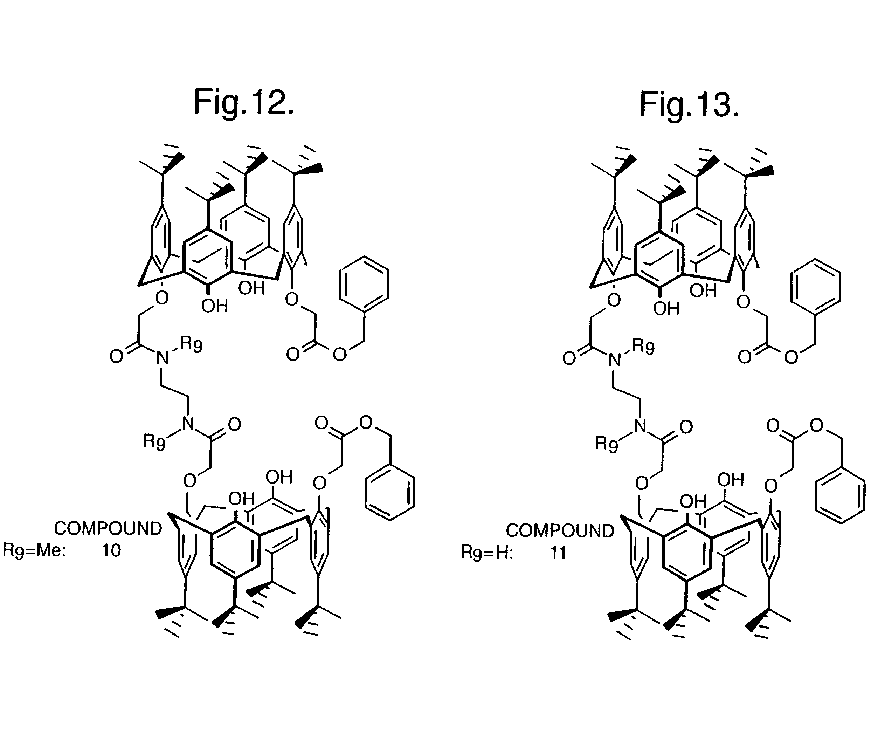

In **Compound 10** (Figure 12) the carboxy group of the calixarenes has been protected with benzyl alcohol. No U extraction occurred at pH 6 (as with Compound 13b). **Compound 11** (Figure 13) is similar to compound 10 but was generated using a different diamine. Again no extraction of U occurred at pH 6. Significant extraction of U and Hg occurred at pH 9 notwithstanding the presence of the protecting group. This implies that a deprotonated carboxy group is not necessary for complexing U or Hg, but that the phenolic groups (deprotonated at high pH) are crucial to extraction. **Compound 11a** is protected with as an ethyl ester, and has the diphenylamine linkage of compound 13b. Again no U extraction occurred at pH 6.

It is clear that the pH dependent specificity of the dimeric compounds above give them utility in the selective extraction of different metals.

EXAMPLE 11- The acid-amide was physisorbed and immobilised onto polystyrene divinyl benzene beads in an inert diluent. Solutions containing U were passed through a chromatography column containing the beads at various different pHs at a flow rate of approximately 2 mls/min. A control experiment was carried out with blank beads. The results are shown Table 2. As can be seen, above pH 2 extraction of U occurred, reaching 100% at pH 3. The kinetics were fast enough to absorb the U from the relatively fast moving mobile phase.

**Table 2**

| pH | Extraction Efficiency | |
|---|---|---|
| | Acid-amide resin | Blank |
| 1 | 2 | 10 |
| 2 | 37 | 34 |
| 3 | 100 | 20 |
| 4 | 100 | 21 |
| 6 | 93 | 21 |
| 9 | 34 | 0 |

EXAMPLE 12 - Synthesis of acid-amide (designated A954 below). Synthetic scheme

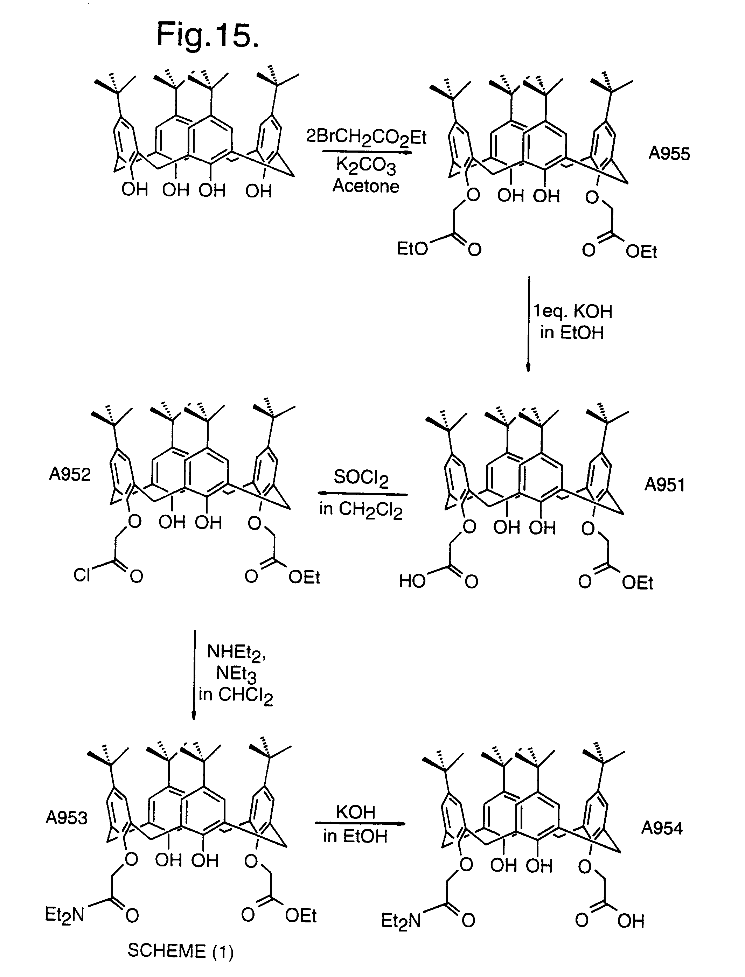

A954 was synthesised using the route shown in Fig 15. The bis-ester(A955) was synthesised following the literature method of Collins et al (1991) J. Chem Soc., Perkin Trans.,1, 3137. Reaction of p-tert-Butylcalix[4]arene with 2 equivalents of ethyl bromoacetate in acetone with potassium carbonate (as base) gave the bis-ester in good yield. This was mono-deprotected using 1 equivalent of potassium hydroxide in ethanol. Although the product contained traces of both bis-ester and bis-acid as impurity, it was used without further purification and the impurities removed in subsequent steps. Overnight reflux with thionyl chloride in dichloromethane gave the acyl chloride which was reacted immediately with excess diethylamine(in dichloromethane with triethylamine present) to give the calixarene amide-ester (A953) in 72% overall yield. Finally, deprotection of the ester group using potassium hydroxide in ethanol gave the desired acid-amide (A954).

### Detailed synthesis

NMR data was compiled after each step, but is shown only for the final product.

A955: p-tert-Butylcalix[4]arene (lOg,0.015mol) and anhydrous potassium carbonate (4.68g, 0.34mol) were slurried in dry acetone (distilled from CaSO₄) for 2 hours. Ethylbromoacetate (5.15g, 0.031mol) was added and the mixture stirred under nitrogen for three days. It was then filtered, the solvent distilled off and the residue dried under vacuum. It was then slurried with cold ethanol to form a white powder and collected by filtration. This solid was washed with a further quantity of cold ethanol and dried under vacuum. Yield 8.97g (73%)

951: bis-ester A955 (8.0g, 9.76mmol) was slurried in ethanol (600m1). Potassium hydroxide (85% AR, 0.55g, 9.76mmol) added and the mixture heated to reflux for 1-2 hours. On cooling the ethanol was reduced in volume (to 50-100ml) and 1 M HCl added to precipitate the product. This was collected by filtration and washed with water(50ml). The product was dried under vacuum.
Yield 6.95g (90%)(Found: C, 73.84; H, 7.42; required C, 75.72; H. 7.62%);

A953: acid-ester A 951 (5.0g, 6.31mmol) was refluxed overnight with thionyl chloride (3.5ml) in dry dichloromethane (100 ml). The solvent was then removed by distillation and the oily yellow residue dried under vacuum. Additions of dichloromethane (4-5ml) were necessary to help azeotrope off the last traces of thionyl chloride. When dry, the product was a glassy off-white solid. The acyl chloride ester was then dissolved in dry dichloromethane (50ml). To this solution was added dropwise, a solution containing dry diethylamine (dried over KOH) (0.98ml, 9.45mmol) and dry triethylamine (dried over CaH₂) (0.87ml, 6.31mmol) in dry dichloromethane (50ml) over 30 minutes. After stirring overnight at room temperature, the solution was transferred to a dropping funnel and washed with 1 M HCl (50ml) and then water (50ml). It was then dried over MgSO4, filtered and the solvent removed in vacuo. The crude product was purified by column chromatography on silica (Kieselgehl) using dichloromethane/methanol(98:2) eluent.
Yield 3.86g (72%) (Found: C, 74.83; H, 8.13; N 2.12. required C,74.87, H, 8.72, N 1.61%)

A 954: amide-ester, A953, (2.20g, 2.48mmol) was dissolved in ethanol (150ml) and potassium hydroxide (0.28g, 4.96mmol) added. The resulting solution was then refluxed for 2 hours. After cooling to room temperature, the volume of the solution was reduced to ca. 25ml by rotary evaporation. Addition of 1 M HCl gave a white precipitate which was collected by filtration and washed with water. It was then dissolved in dichloromethane (30ml). washed with 1 M HCl (30ml).water (30ml) and then dried over MgSO4. The solvent was removed in vacuo to give a foamy white solid. It was converted to a powder by dissolving in a minimum of dichloromethane and adding hexane (30-40ml)-evaporation to dryness gave a white solid. Yield 2.06g(97%) (Found: C, 75.24; H, 8.77; N 1.97. required C, 75.33, H,8.51, N 1.69%).
NMR data (300MHz, CDCl3) 1.07 (9H, s, -Bu). 1.11 (9H, s, -Bu), 1.25(18H, s, -Bu), 1.25 (3H, t, -CH₃), 3.38 (2H, d, Ar-CH₂-Ar), 3.38 (2H,q, -NCH₂-), 3.42 (2H, d, J=13.0Hz, Ar-CH₂-Ar), 3.55 (2H, q, -NCH₂-),4.22 (2H, d, J=13.0Hz, Ar-CH₂-Ar), 4.30 (2H, d, J=13.3Hz, Ar-CH₂-Ar).4.64 (2H, s, -OCH₂CO-) , 4.78 (2H, s, -OCH₂CO-), 6.93 (2H, s, Ar),6.99 (2H, s, Ar), 7.03 (2H, d, Ar), 7.06 (2H, d, Ar), 8.90 (2H, br s,-OH); (75.42MHz, CDCl3) 13.02, 14.36, 31.12, 31.68, 32.17, 32.35, 33.91, 34.05, 34.16, 40.78, 41.20, 72.44, 73.29, 125.24, 125.55, 126.10, 127.21, 128.29, 132.71, 132.94, 142.32, 147.49, 148.51, 149.76, 150.11, 150.21, 166.71, 170.44; FAB m.s., m/z 864 (M+2Na⁺-H., 18%), 842 (M+Na⁺, 100), 820 (M+, 10).

It should be noted that the synthesis of other calixarenes falling within the claims of the present application may be readily achieved by the skilled person in the light of the disclosure of the present document, particularly in combination with the common general knowledge of the skilled person, as evidenced for example by the teaching and references of EP 0 432 989.

### A 954/metal complex synthesis

To prepare Ln(NO₃)₃.nDMSO, n=3,4 Ln₂O₅ was dissolved in a minimum of nitric acid (fast exothermic process for large Ln, slow process for small Ln). To the resulting solution was added a 5-6 fold excess of dimethyl sulphoxide. Ethanol and then diethyl ether were then added to precipitate the product. Occasionally, when the product oiled out, it was necessary to decant the mother liquor, add more ethanol/diethyl ether and then scratch with a glass rod. The product was then collected by filtration, redissolved in DMSO and precipitated with ethanol/ether. The final product was collected by filtration and dried under vacuum. All DMSO solvates gave elemental analyses in accordance with their proposed structures

A simpler method involved the use of Ln(NO₃)₃ penta and hexahydrates instead of the oxide. In this case the salt was twice dissolved in DMSO and precipitated with ethanol and diethyl ether.

The calixarene acid-amide A954 (0.0189g, 0.023mmol) was dissolved in 1ml DMF. To this solution was added Ln(NO₃)₃.nDMSO (n=3 or 4, 0.025mmol) also in 1 ml DMF. After the further addition of 30 microlitres of triethylamine (excess), the solution was immediately filtered and left to stand. As mentioned earlier. the larger lanthanides precipitated quite quickly from solution whereas the smaller ones took considerably longer. The precipitated complex was then collected by filtration and washed with a minimum of cold ethanol (ca. 0.5ml) and dried under vacuum. Attempts were made to recrystallise these complexes from dichloromethane/ethanol. This typically involved dissolving the complex in dichloromethane (1.5ml) and then adding ethanol (1 ml). After filtering, the solution was left to slowly evaporate.

For the larger lanthanides (La-Eu), the complex precipitated fairly quickly from solution, and was then recrystallised from dichloromethane/ethanol. In case of the Eu and Sm complexes, crystals suitable for X-ray crystallographic analysis were isolated.

Precipitated from DMF/NEt:Sm complex of A954; Found: C, 64.0; H, 7.2; N 3.3. required C, 64.3, H, 7.5, N 3.7%
Eu complex of A954; Found: C, 63.9; H, 7.1; N 3.4. required C, 64.2, H, 7.5, N 3.7%

Recrystallised from ethanol/dichloromethane:
Eu complex of A954; Found: C, 65.6; H, 7.5; N 2.8. required C, 65.5, H, 7.9, N 2.6%

The smaller lanthanide complexes (Lu) less readily precipitated from DMF solution than the larger ones described above, instead crystallising out only after a period of weeks.

EXAMPLE 13 - Synthesis of azacrown-acid calix[4]arenes

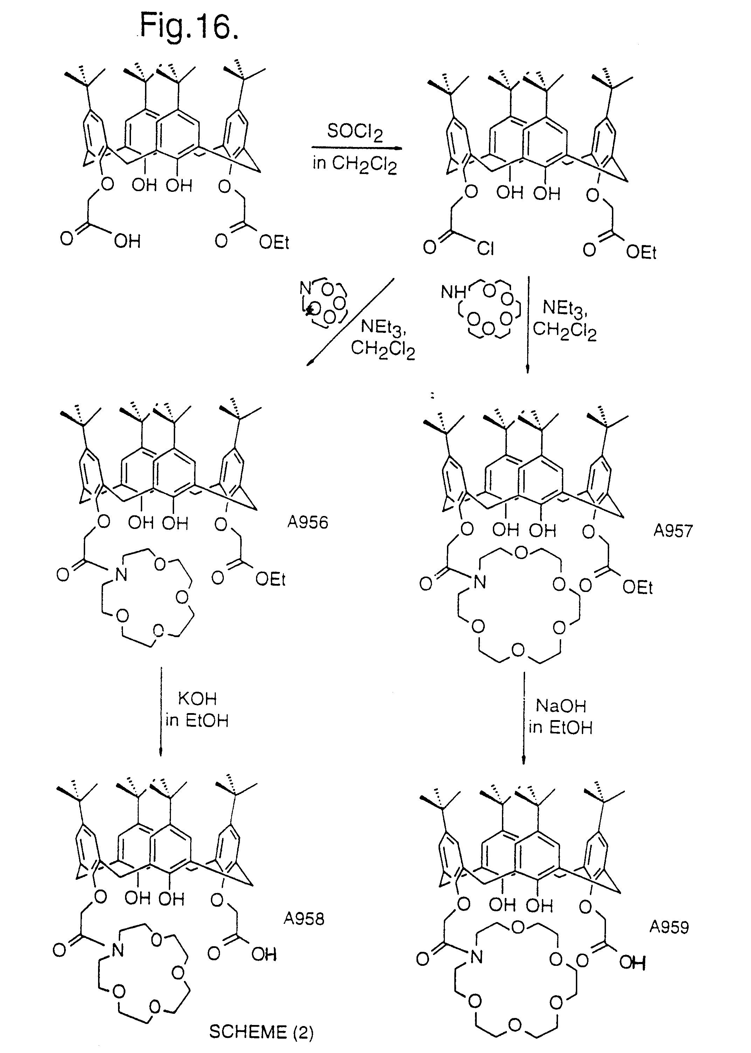

In attempt to form discrete monomeric complexes across the Lanthanide series, the azacrown-acid calix[4]arenes A958 and A959 (Fig 16) were prepared with the idea that the extra O-donor sites present would more easily satisfy the normal 8-10 coordination sphere of the larger Lanthanides.

The synthetic scheme used in the synthesis of the simpler acid-amide (A954) was also applied in the synthesis of the azacrownacidcalix[4]arenes, Fig 15. For the final deprotection step, in order to eliminate the possibility of isolating alkalimetal complexes of the product , potassium hydroxide was used as base in the deprotection of the N-aza-15-crown-5 ligand and sodium hydroxide in the case of the N-aza-18-crown-6 ligand.

### Isolation of complexes

Preliminary work was also begun on the isolation of the Lanthanide complexes of these ligands, the majority of this involving the N-aza-15-crown-5 analogue only. The same methods were applied as for the simpler acid-amide (A954) and, in general, the same observations made. Again the larger Ln cations formed complexes which readily precipitated from DMF solution. Attempts at recrystallisation of these complexes from dichloromethane/ethanol again yielded X-ray crystallographic quality crystals of the Sm complex. Disappointingly, however, the anticipated monomeric complex was not formed. Instead, a similar dimeric structure was adopted with the aza-crown folding away and not coordinating to Sm.

EXAMPLE 14 - U.V. Spectra

The observed maxima for the A 954 acid-amide are listed in Table 3 together with the corresponding values for selected complexes. The extinction values given are approximate only. Given that the sample sizes measured were only about 1 mg, weighing errors could easily account for apparent differences in absorption between related species.

EXAMPLE 15 - Synthesis of acid-amide dimer. The dimers of Example 10 were prepared by methods analogous to those above. In the case of 11a, compound A952 (Fig 15) was prepared as described above. Two molecules of A952 were dimerised with m-phenylenediamine in dichloromethane and triethylamine. The yield was 68%. Compound 11b was prepared from 11a by regenerating the carboxy group with potassium hydroxide in ethanol. The yield was 90%. The other dimers were prepared using different diamines (e.g. 1,2-di(methylamino)ethane for 11 and 13b). Other protecting groups can be added either as alcohols to the deprotected acid group, or incorporated into the precursor e.g. by substituting the ethylbromoacetate used to prepare A955 in Example 12 with a bromylated benzyl ester. The diamine synthetic route is flexible in that a wide variety of spacer groups may be introduced between the calixarene halves, allowing factors such as chain length, coordination etc. to be assessed.

**Table 3**

| Compound/complex | Wavelength | Maxima |
|---|---|---|
| | (nm) | cm⁻¹M⁻¹ |
| 954 | 228 | 36000 |
| | 282 | 9500 |
| 954/La | 228 | 50000 |
| | 260(sh) | 15000 |
| | 307 | 9400 |
| 954/Sm | 228 | 46000 |
| | 260(sh) | 13000 |
| | 307 | 9600 |
| 954/Eu | 228 | 48000 |
| | 260(sh) | 15000 |
| | 306 | 9700 |

## Claims

1. Calixarenes of the formula (I) 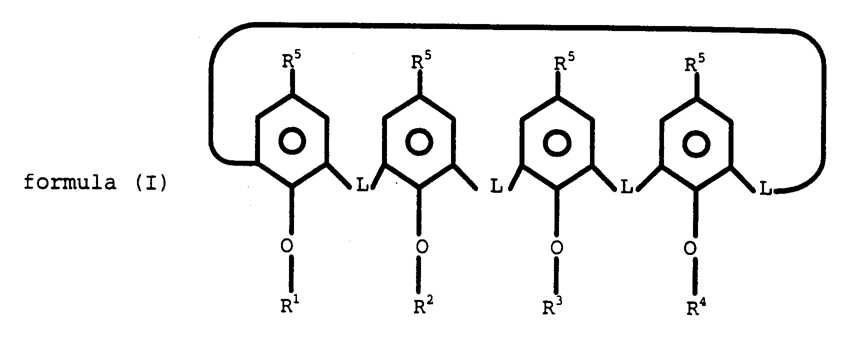 wherein:
L is [ -CH₂- ] or [ -O-CH₂-O- ] and may be the same or different between each aryl group.
R⁵ is H, halogen, or C₁ - C₁₀ aliphatic hydrocarbyl group, C₆ - C₂₀ aryl group, C₆ - C₂₀ hydrocarbylaryl group, any of which may optionally be substituted by one or more halo or oxo groups or interrupted by one or more oxo groups, and R⁵ may be the same of different on each aryl group.
R¹ comprises a carboxy group which may or may not be protonated or protected.
two groups out of R², R³, and R⁴ are H
the one group out of R², R³, and R⁴ not being H comprises an amide group

2. Calixarenes as claimed in claim 1 wherein R² and R⁴ are H and R³ comprises amide group.

3. Calixarenes as claimed in claim 1 or claim 2 wherein L is [-CH₂-] between each of the aryl groups.

4. Calixarenes as claimed in any one of claims 1 to 3 wherein R⁵ is tertiary butyl.

5. Calixarenes as claimed in any one of claims 1 to 4 wherein the carboxy group R¹ conforms to the general formula (A):
(A) [ -X-COOR¹⁰]
wherein X is a C₁, a C₂ or a C₃ carbon chain being a part of an aliphatic hydrocarbyl group, aryl group or hydrocarbylaryl group, any of which may optionally be substituted by one or more halo, oxo or nitro groups; and R¹⁰ is H or a protecting group being a salt or an Ester derivative.

6. Calixarenes as claimed in claim 5 wherein R¹⁰ is H and the aliphatic hydrocarbyl group, aryl group or hydrocarbylaryl group of formula (A) are substituted by one or more groups which cause a reduction in the pKa of the carboxylic acid group with respect to the unsubstituted molecule.

7. Calixarenes as claimed in claim 5 wherein R¹ is of the general formula (B):
(B) [ - (C.R⁶.R⁷)ₙ-COOR¹⁰]
wherein n is 1, 2 or 3 and R⁶ and R⁷ are H or halogen and can be the same or different on each carbon.

8. Calixarenes as claimed in claim 5 wherein R¹ is of the general formula (C): 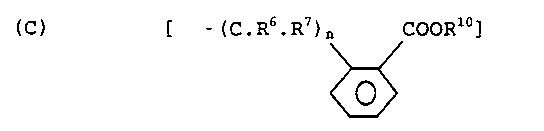 wherein n is 0 or 1 and R⁶ and R⁷ are H or halogen and can be the same or different on each carbon and wherein the phenyl ring of the benzoic acid group may be optionally substituted by one or more halo, oxo or nitro groups

9. Calixarenes as claimed in claim 8 wherein R¹⁰ is H and the phenyl ring of the benzoic acid of formula (C) is substituted by one or more groups which cause a reduction in the pKa of the carboxy group with respect to the unsubstituted molecule.

10. Calixarenes as claimed in any one of claims 5 to 9 wherein n is 1 and R⁶ and R⁷ are both H.

11. Calixarenes as claimed in any one of the preceding claims wherein the amide group R², R³ or R⁴ of formula (I) is of the general formula (D): 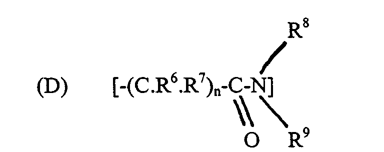 wherein n is 1, 2 or 3 and R⁶ and R⁷ are H, halogen or C₁₋₁₀ aliphatic hydrocarbyl group, and can be the same or different on each carbon, and wherein R⁸ and R⁹, which may be the same or different, are H or C₁₋₁₀ aliphatic hydrocarbyl group which may be substituted by one or more halo groups, or may be a cycloaliphatic ring formed by R⁸ and R⁹ together, or may be conjugated to a second calixarene.

12. A calixarene of the formula (II) which is 5, 11, 17, 23-tetra-tert-butyl-25-[hydroxycarbomylmethoxy]-27-[(N-diethylamino)carbomylmethoxy]-26-28-dihydroxycalix[4]arene.

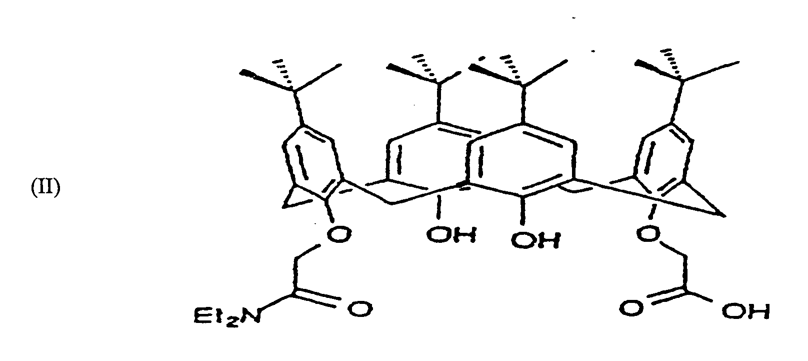

13. Calixarenes as claimed in any one of the preceding claims wherein some or all of phenyl groups of the calixarene ring are further peripherally substituted.

14. A method of sequestering metals comprising contacting the metals with a calixarene as claimed in any one of the preceding claims.

15. A method as claimed in claim 14, wherein the method is carried out at a pH of between 2 and 11.

16. A method as claimed in claim 14 or 15 wherein the pH at which the method is carried out is buffered.

17. A method as claimed in claim 16 wherein the buffer used is citrate.

18. A method as claimed in any one of claims 14 to 17 comprising the following steps:
(i) dissolving the calixarene in an hydrophobic organic solvent;
(ii) mixing the organic solvent with an aqueous phase containing metal ions;
(iii) agitating the organic solvent and aqueous phase together;
(iv) recovering the metal from the organic phase.

19. A method as claimed in any one of claims 14 to 18 wherein the metal is selected from the list: a Lanthanide, U, Hg, Am, Pb, Sr, Bi, Y.

20. A calixarene as claimed in any one of claims 1 to 13 further characterised in that the calixarene is solid phase bound.

21. A process for preparing a calixarene as claimed in any one of claims 1 to 13 comprising the sequential steps of:
(i) bis-esterification of a calix[4]arene;
(ii) deprotection of a first ester group to form a first acid group;
(iii) chlorination of the said first acid group to form an acyl chloride; and
(iv) substitution of the chlorine group in the said acyl chloride with a diamine moiety;

22. A process as claimed in claim 21 wherein the process comprises a subsequent step of the deprotection of the second ester group to form an acid moiety.

23. A calixarene dimer comprising a calixarene as claimed in claim 11 wherein one of the R⁸ and R⁹ groups is conjugated to a second calixarene.

24. A dimer as claimed in claim 23 comprising two calixarenes as claimed in claim 11 wherein the R⁸ or R⁹ group of one calixarene is conjugated to the R⁸ or R⁹ group of the other calixarene, optionally through a spacer group R¹¹, the optional spacer group R¹¹ being C₁ - C₆ aliphatic hydrocarbyl group, C₆- C₁₀ aryl group, C₆ - C₁₆ hydrocarbylaryl group any of which may optionally be substituted by one or more halo or oxo groups or interrupted by one or more oxo groups.

25. A dimer as claimed in claim 24 wherein there is a 1, 2, 3 or 4 atom chain between the nitrogen atoms of the two amide groups.

26. A process for preparing a dimer as claimed in any one of claims 23 to 25 comprising the use of a diamine to conjugate two calixarene molecules.

27. A process for preparing a dimer as claimed in claim 26 wherein two equivalents of a calixarene bearing an acyl chloride substituent are reacted with one equivalent of diamine.

## Patentansprüche

1. Calixarene der Formel (I) 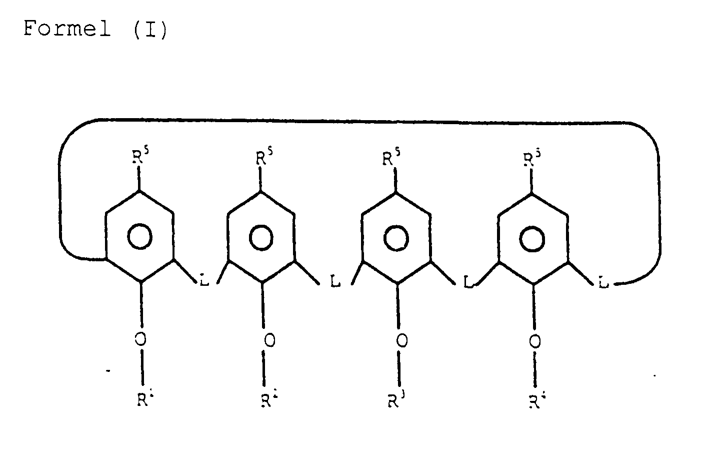 wobei gilt:
L ist [-CH₂-] oder [-O-CH₂-O-] und kann zwischen jeder Arylgruppe gleich oder verschieden sein,
R⁵ ist H, Halogen oder eine aliphatische C₁-C₁₀-Hydrocarbylgruppe, C₆-C₂₀-Arylgruppe, C₆-C₂₀-Hydrocarbylarylgruppe, die jeweils gegebenenfalls mit einer oder mehreren Halogen- oder Oxogruppen substituiert oder durch eine oder mehrere Oxogruppen unterbrochen sein können, und R⁵ kann für jede Arylgruppe gleich oder verschieden sein,
R¹ umfaßt eine Carboxygruppe, die protoniert oder geschützt sein kann oder nicht,
zwei von den Gruppen R², R³ und R⁴ sind H,
wobei die eine Gruppe von R², R³ und R⁴, die kein H ist, eine Amidgruppe umfaßt.

2. Calixarene nach Anspruch 1, wobei R² und R⁴ H sind und R³ eine Amidgruppe umfaßt.

3. Calixarene nach Anspruch 1 oder Anspruch 2, wobei L zwischen jeder der Arylgruppen [-CH₂-] ist.

4. Calixarene nach einem der Ansprüche 1 bis 3, wobei R⁵ tert-Butyl ist.

5. Calixarene nach einem der Ansprüche 1 bis 4, wobei die Carboxygruppe R¹ die allgemeine Formel (A) hat:
(A) [ -X-COOR¹⁰]
wobei X eine C₁-, eine C₂- oder eine C₃-Kohlenstoffkette als Teil einer aliphatischen Hydrocarbylgruppe, Arylgruppe oder Hydrocarbylarylgruppe ist, die jeweils gegebenenfalls mit einer oder mehreren Halogen-, Oxo- oder Nitrogruppen substituiert sein kann, und R¹⁰ H oder eine Schutzgruppe in Form eines Salzes oder eines Ether-Derivats ist.

6. Calixarene nach Anspruch 5, wobei R¹⁰ H ist und die aliphatische Hydrocarbylgruppe, Arylgruppe oder Hydrocarbylarylgruppe der Formel (A) mit einer oder mehreren Gruppen substituiert ist, die den pKa-Wert der Carbonsäuregruppe in bezug auf das unsubstituierte Molekül erniedrigen.

7. Calixarene nach Anspruch 5, wobei R¹ die allgemeine Formel (B) hat:
(B) [ - (C.R⁶.R⁷)ₙ-COOR¹⁰]
wobei n 1, 2 oder 3 ist und R⁶ und R⁷ H oder Halogen sind und für jedes Kohlenstoffatom gleich oder verschieden sein können.

8. Calixarene nach Anspruch 5, wobei R¹ die allgemeine Formel (C) hat: 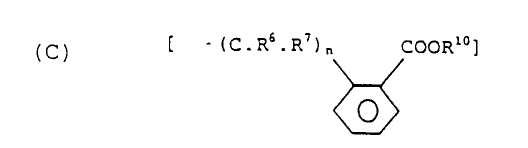 wobei n 0 oder 1 ist und R⁶ und R⁷ H oder Halogen sind und für jedes Kohlenstoffatom gleich oder verschieden sein können und der Phenylring der Benzoesäuregruppe gegebenenfalls mit einer oder mehreren Halogen-, Oxo- oder Nitrogruppen substituiert sein kann.

9. Calixarene nach Anspruch 8, wobei R¹⁰ H ist und der Phenylring der Benzoesäure von Formel (C) mit einer oder mehreren Gruppen substituiert ist, die den pKa-Wert der Carboxygruppe in bezug auf das unsubstituierte Molekül erniedrigen.

10. Calixarene nach einem der Ansprüche 5 bis 9, wobei n 1 ist und R⁶ und R⁷ beide H sind.

11. Calixarene nach einem der vorhergehenden Ansprüche, wobei die Amidgruppe R², R³ oder R⁴ der Formel (I) die allgemeine Formel (D) hat: 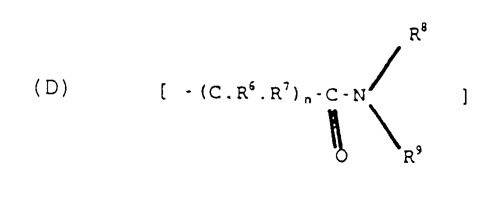 wobei n 1, 2 oder 3 ist und R⁶ und R⁷ H, Halogen oder eine aliphatische C₁-C₁₀-Hydrocarbylgruppe sind und für jedes Kohlenstoffatom gleich oder voneinander verschieden sein können und R⁸ und R⁹, die gleich oder voneinander verschieden sein können, H oder eine aliphatische C₁-C₁₀-Hydroxycarbylgruppe sind, die mit einer oder mehreren Halogengruppen substituiert sein kann, oder R⁸ und R⁹ zusammen einen cycloaliphatischen Ring bilden oder an ein zweites Calixaren konjugiert sein können.

12. Calixaren der Formel (II), nämlich 5,11,17,23-Tetra-tert-butyl-25-[hydroxycarbomylmethoxy]-27-[(N-diethylamino)carbomylmethoxy]-26-28-dihydroxycalix[4]aren

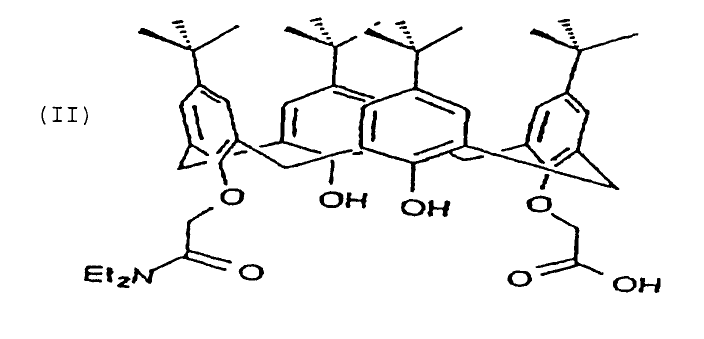

13. Calixarene nach einem der vorhergehenden Ansprüche, wobei einige oder sämtliche Phenylgruppen des Calixaren-Rings ferner am Rand substituiert sind.

14. Verfahren zur Komplexierung von Metallen, bei dem die Metalle mit einem Calixaren nach einem der vorhergehenden Ansprüche in Kontakt gebracht werden.

15. Verfahren nach Anspruch 14, wobei das Verfahren bei einem pH-Wert zwischen 2 und 11 durchgeführt wird.

16. Verfahren nach Anspruch 14 oder 15, wobei der pH-Wert, bei dem das Verfahren durchgeführt wird, gepuffert ist.

17. Verfahren nach Anspruch 16, wobei der verwendete Puffer Citrat ist.

18. Verfahren nach einem der Ansprüche 14 bis 17, bei dem:
(i) das Calixaren in einem hydrophoben organischen Lösungsmittel gelöst wird,
(ii) das organische Lösungsmittel mit einer Metallionen enthaltenden wäßrigen Phase vermischt wird,
(iii) das organische Lösungsmittel und die wäßrige Phase zusammen umgewälzt werden,
(iv) das Metall aus der organischen Phase gewonnen wird.

19. Verfahren nach einem der Ansprüche 14 bis 18, wobei das Metall unter einem Lanthanid, U, Hg, Am, Pb, Sr, Bi, Y ausgewählt ist.

20. Calixaren nach einem der Ansprüche 1 bis 13, außerdem dadurch gekennzeichnet, daß das Calixaren an eine feste Phase gebunden ist.

21. Verfahren zur Herstellung eines Calixarens nach einem der Ansprüche 1 bis 13, bei dem:
(i) ein Calix[4]aren zweifach verestert wird,
(ii) eine erste Estergruppe zur Bildung einer ersten Säuregruppe von der Schutzgruppe befreit wird,
(iii) die erste Säuregruppe unter Bildung eines Acylchlorids chloriert wird und
(iv) die Chlorgruppe in dem Acylchlorid durch einen Diamin-Rest substituiert wird.

22. Verfahren nach Anspruch 21, wobei bei dem Verfahren als sich anschließender Schritt die zweite Estergruppe unter Bildung eines Säure-Restes von der Schutzgruppe befreit wird.

23. Calixaren-Dimer, das ein Calixaren nach Anspruch 11 enthält, wobei eine der Gruppen R⁸ und R⁹ an ein zweites Calixaren konjugiert ist.

24. Dimer nach Anspruch 23, das zwei Calixarene nach Anspruch 11 enthält, wobei die Gruppe R⁸ oder R⁹ eines Calixarens an die Gruppe R⁸ oder R⁹ des anderen Calixarens konjugiert ist, und zwar gegebenenfalls über eine Abstandsgruppe R¹¹, wobei die fakultative Abstandsgruppe R¹¹ eine aliphatische C₁-C₆-Hydrocarbylgruppe, C₆-C₁₀-Arylgruppe, C₆-C₁₆-Hydrocarbylarylgruppe ist, die jeweils gegebenenfalls mit einer oder mehreren Halogen- oder Oxogruppen substituiert oder durch eine oder mehrere Oxogruppen unterbrochen sein können.

25. Dimer nach Anspruch 24, wobei sich eine Kette mit 1, 2, 3 oder 4 Atomen zwischen den Stickstoffatomen der zwei Amidgruppen befindet.

26. Verfahren zur Herstellung eines Dimers nach einem der Ansprüche 23 bis 25, bei dem ein Diamin zur Konjugation von zwei Calixarenmolekülen verwendet wird.

27. Verfahren zur Herstellung eines Dimers nach Anspruch 26, wobei zwei Äquivalente eines einen Acylchlorid-Substituenten tragenden Calixarens mit einem Äquivalent eines Diamins umgesetzt werden.

## Revendications

1. Calixarènes de formule (I) 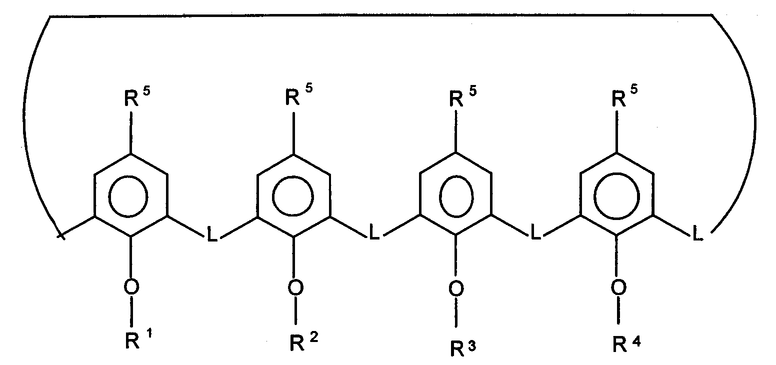 dans laquelle
L est [-CH₂-] ou [-O-CH₂-O-] et peut être identique ou différent d'un groupe aryle à l'autre,
R⁵ représente un atome d'hydrogène, d'halogène, ou un groupe hydrocarbyle aliphatique en C₁-C₁₀, un groupe aryle en C₆-C₂₀, un groupe hydrocarbylaryle en C₆-C₂₀, dont l'un quelconque peut être éventuellement substitué par un ou plusieurs groupes halogéno ou oxo ou être interrompu par un ou plusieurs groupes oxo, et R⁵ peut être identique ou différent d'un groupe aryle à l'autre,
R¹ comprend un groupe carboxy qui peut être ou non protoné ou protégé,
deux groupes parmi R², R³ et R⁴ sont H,
l'un des groupes parmi R², R³ et R⁴ n'étant pas H comprend un groupe amide.

2. Calixarènes selon la revendication 1, dans lesquels R² et R⁴ sont H et R³ comprend un groupe amide.

3. Calixarènes selon la revendication 1 ou la revendication 2, dans lesquels L est [-CH₂-] entre chacun des groupes aryle.

4. Calixarènes selon l'une quelconque des revendications 1 à 3, dans lesquels R⁵ est un groupe tert-butyle.

5. Calixarènes selon l'une quelconque des revendications 1 à 4, dans lesquels le groupe carboxy R¹ répond à la formule générale (A) :
(A) [-X-COOR¹⁰]
dans laquelle X est une chaîne carbonée en C₁, en C₂ ou en C₃ faisant partie d'un groupe hydrocarbyle aliphatique, d'un groupe aryle ou d'un groupe hydrocarbylaryle, dont l'un quelconque peut être éventuellement substitué par un ou plusieurs groupes halogéno, oxo ou nitro ; et R¹⁰ est H ou un groupe protecteur qui est un sel ou un dérivé d'ester.

6. Calixarènes selon la revendication 5, dans lesquels R¹⁰ est H et le groupe hydrocarbyle aliphatique, le groupe aryle ou le groupe hydrocarbylaryle de formule (A) est substitué par un ou plusieurs groupes qui entraînent une réduction du pKa du groupe acide carboxylique par rapport à celui de la molécule non substituée.

7. Calixarènes selon la revendication 5, dans lesquels R¹ répond à la formule (B) :
(B) [-(C.R⁶.R⁷)ₙ-COOR¹⁰]
dans laquelle n vaut 1, 2 ou 3 et R⁶ et R⁷ sont un atome d'hydrogène ou d'halogène et peuvent être identiques ou différents sur chaque carbone.

8. Calixarènes selon la revendication 5, dans lesquels R¹ répond à la formule générale (C) : 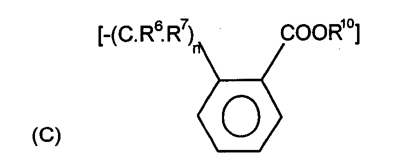 dans laquelle n vaut 0 ou 1 et R⁶ et R⁷ sont un atome d'hydrogène ou d'halogène et peuvent être identiques ou différents sur chaque carbone et dans lesquels le noyau phényle du groupe acide benzoïque peut être éventuellement substitué par un ou plusieurs groupes halogéno, oxo ou nitro.

9. Calixarènes selon la revendication 8, dans lesquels R¹⁰ est H et le noyau phényle de l'acide benzoïque de formule (C) est par un ou plusieurs groupes qui entraînent une réduction du pKa du groupe carboxy par rapport à celui de la molécule non substituée.

10. Calixarènes selon l'une quelconque des revendications 5 à 9, dans lesquels n vaut 1 et R⁶ et R⁷ sont tous deux H.

11. Calixarènes selon l'une quelconque des revendications précédentes, dans lesquels le groupe amide R², R³ ou R⁴ de formule (I) répond à la formule générale (D) : 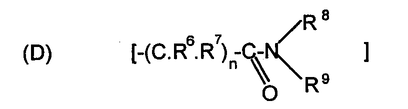 dans laquelle n vaut 1, 2 ou 3, et R⁶ et R⁷ sont un atome d'hydrogène ou d'halogène, ou un groupe hydrocarbyle aliphatique en C₁-C₁₀, et peuvent être identiques ou différents sur chaque carbone, et dans laquelle R⁸ et R⁹, qui peuvent être identiques ou différents, sont H ou un groupe hydrocarbyle aliphatique en C₁-C₁₀, qui peut être substitué par un ou plusieurs groupes halogéno, ou qui peut être un noyau cycloaliphatique formé par R⁸ et R⁹ ensemble, ou peuvent être conjugués à un deuxième calixarène.

12. Calixarène de formule (II) qui est le 5,11,17,23-tétra-tert-butyl-25-[hydroxycarbomylméthoxy]-27-[(N-diéthylamino)carbomylméthoxy]-26,28-dihydroxycalix[4]arène.

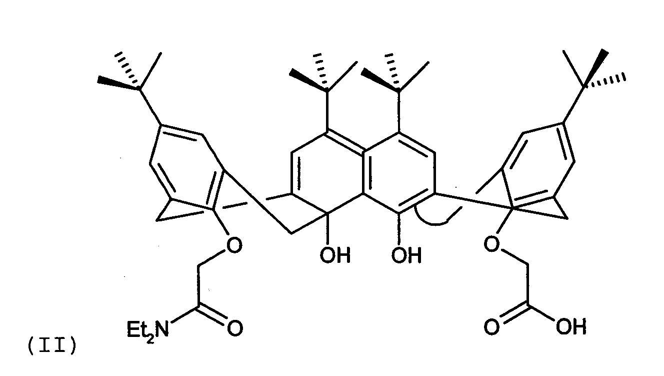

13. Calixarènes selon l'une quelconque des revendications précédentes, dans lesquels une partie ou tous les groupes phényle du noyau calixarène sont en outre substitués à la périphérie.

14. Procédé de séquestration de métaux comprenant la mise en contact des métaux avec un calixarène selon l'une quelconque des revendications précédentes.

15. Procédé selon la revendication 14, dans lequel le procédé est réalisé à un pH compris entre 2 et 11.

16. Procédé selon la revendication 14 ou 15, dans lequel le pH auquel le procédé est réalisé est tamponné.

17. Procédé selon la revendication 16, dans lequel le tampon utilisé est un citrate.

18. Procédé selon l'une quelconque des revendications 14 à 17, comprenant les étapes suivantes consistant à :
(i) dissoudre le calixarène dans un solvant organique hydrophobe ;
(ii) mélanger le solvant organique avec une phase aqueuse contenant des ions métalliques ;
(iii) agiter le solvant organique et le phase aqueuse ensemble ;
(iv) récupérer le métal de la phase organique.

19. Procédé selon l'une quelconque des revendications 14 à 18, dans lequel le métal est choisi dans la liste : un lanthanide, U, Hg, Am, Pb, Sr, Bi, Y.

20. Calixarène selon l'une quelconque des revendications 1 à 13, caractérisé en outre en ce que le calixarène est lié à une phase solide.

21. Procédé de préparation d'un calixarène selon l'une quelconque des revendications 1 à 13, comprenant les étapes séquentielles de :
(i) une bis-estérification d'un calix[4]arène ;
(ii) une déprotection d'un premier groupe ester pour former un premier groupe acide ;
(iii) une chloration dudit premier groupe acide pour former un chlorure d'acyle ; et
(iv) un remplacement du groupe chlore dans ledit chlorure d'acyle par un groupement diamine.

22. Procédé selon la revendication 21, dans lequel le procédé comprend une étape ultérieure de déprotection du deuxième groupe ester pour former un groupement acide.

23. Dimère de calixarène comprenant un calixarène selon la revendication 11, dans lequel l'un des groupes R⁸ et R⁹ est conjugué à un deuxième calixarène.

24. Dimère selon la revendication 23, comprenant deux calixarènes selon la revendication 11, dans lesquels le groupe R⁸ ou R⁹ de l'un des calixarènes est conjugué au groupe R⁸ ou R⁹ de l'autre calixarène, éventuellement par l'intermédiaire d'un groupe écarteur R¹¹, le groupe écarteur R¹¹ facultatif étant un groupe hydrocarbyle aliphatique en C₁-C₆, un groupe aryle en C₆-C₁₀, un groupe hydrocarbylaryle en C₆-C₁₆, dont l'un quelconque peut être éventuellement substitué par un ou plusieurs groupes halogéno ou oxo ou interrompu par un ou plusieurs groupes oxo.

25. Dimère selon la revendication 24, dans lequel une chaîne à 1, 2, 3 ou 4 atomes est située entre les atomes d'azote des deux groupes amide.

26. Procédé de préparation d'un dimère selon l'une quelconque des revendication 23 à 25, comprenant l'utilisation d'une diamine pour conjuguer deux molécules de calixarène.

27. Procédé de préparation d'un dimère selon la revendication 26, dans lequel deux équivalents d'un calixarène portant un substituant chlorure d'acyle sont mis à réagir avec un équivalent de diamine.
